Europäisches Patentamt

European Patent Office

Office européen des brevets

(11) Numéro de publication: **0 152 717**
**A1**

# (12) DEMANDE DE BREVET EUROPEEN

(21) Numéro de dépôt: **84450024.9**

(22) Date de dépôt: **09.11.84**

(51) Int. Cl.⁴: **A 61 M 1/36**

(30) Priorité: **14.11.83 FR 8318164**

(43) Date de publication de la demande: **28.08.85**
**Bulletin 85/35**

(84) Etats contractants désignés: **AT BE CH DE FR GB IT LI
LU NL SE**

(71) Demandeur: **SOCIETE SANTE TECHNIQUE ET
RECHERCHE S.A.R.L., 5 Boulevard Charles Bourseult,
F-13014 Marseille (FR)**

(72) Inventeur: **Issautier, Roland, 108 Rue de la Farigoule,
F-34100 Montpellier (FR)**

(74) Mandataire: **Ravina, Bernard, Cabinet Bernard
RAVINA 24, boulevard Riquet, F-31000 Toulouse (FR)**

(54) **Appareil d'hémodialyse.**

(57) L'appareil d'hémodialyse selon la présente invention est du type comportant un dialyseur (1) à membrane dans lequel circule le sang et un liquide de dialyse qui entraîne les impuretés transgressant au travers de la membrane, un réservoir (3) de liquide de dialyse alimentant le dialyseur, une cartouche (2) régénérante étant placée sur le circuit de retour entre le dialyseur (1) et le réservoir (3), ledit appareil étant caractérisé en ce que le dialyseur, la cartouche et le réservoir sont groupés en un même ensemble séparable des moniteurs d'assistance circulatoire.

APPAREIL D'HEMODIALYSE

La présente invention a pour objet un appareil d'hémodialyse.

L'hémodialyse est une technique utilisée pour retirer périodiquement du sang les impuretés qui peuvent s'y trouver, soit en raison d'insuffisances rénales, soit en raison d'intoxications.

La technique connue fait usage d'un dispositif dialyseur constitué schématiquement par une enceinte divisée en une chambre de circulation du sang envoyé par une pompe et en une chambre de circulation d'un liquide de dialyse avec pompe de circulation.

Les dites chambres sont séparées par une membrane réalisée de toute manière connue, qui est perméable ou semi-perméable et qui est conçue pour permettre la transgression de la chambre de circulation du sang à celle de circulation du liquide de dialyse des impuretés de poids molléculaire déterminé.

Après passage dans le dialyseur, le liquide dialysat doit être remplacé, cela suppose l'utilisation d'un réservoir de grande capacité puisqu'il est courant pour une dialyse de 6 heures d'utiliser de 200 à 100 litres de liquide ou l'utilisation d'un générateur fabriquant en continu le liquide de dialyse.

On a proposé dans des matériels perfectionnés d'intercaler entre la chambre de passage du liquide de dialysat et le réservoir de liquide une cartouche absorbante jetable après usage, constituée par exemple de charbon actif et de résines échangeuses d'ions, qui a pour effet de régénérer le dialysat.

Cette solution permet d'obtenir une réduction d'encombrement de l'appareil.

L'utilisation de cartouche de régénération entraîne cependant un appauvrissement du liquide régénéré en certains de ses constituants, tels que par exemple le calcium, le magnésium et le potassium et un enrichissement en certains autres composants, tels que le sodium. Cela suppose le recours à des moyens permettant de maintenir un équilibre constant de la composition du liquide de dialyse par exemple par adjonction d'un infusat.

Les matériels connus de ce type se définissent donc comme composé d'un dialyseur tel que décrit plus haut alimenté par un circuit à sang au moyen d'une pompe et d'un circuit de liquide de dialyse avec une pompe forçant le produit dialysat à passer sous pression au travers de la cartouche et d'un réservoir dans lequel le liquide est équilibré par adjonction des composants nécessaires et réchauffé éventuellement par des moyens appropriés et est brassé éventuellement par recirculation avant retour vers le dialyseur.

Ces appareils sont donc en dépit de l'avantage apporté par l'utilisation de la cartouche jetable de conception complexe et de fabrication couteûse.

Une recherche dans le sens de la simplification a été effectuée par

3

0152717

réalisation, comme décrit dans le brevet FR 74.31591, d'un ensemble combiné de dialyseur et d'absorbeur regroupé dans un manchon où l'un entoure l'autre, le dit manchon pouvant être immergé directement dans le réservoir de liquide dialysat comme décrit dans la demande de brevet N° 74.31594 du même demandeur.

Le fait que le manchon dialyseur absorbeur soit jetable après usage n'apporte pas de solution fondamentale à la complexité de ces matériels qui nécessitent par ailleurs l'ensemble des éléments constitutifs mentionnés plus haut.

Leur mise en oeuvre qui nécessite entre autre un rinçage avant chaque séance et une stérilisation après chaque séance et leur coût de fabrication en fait des appareils dont l'utilisation n'est possible qu'à poste fixe par un personnel ayant reçu la formation nécessaire.

Le malade utilisateur est donc lié au lieu de localisation de l'appareil ce qui réduit sa liberté de mouvement.
Cela est contraire à la tendance actuelle visant à la plus grande autonomie possible des malades.

La présente invention vise à réaliser un appareil d'hémodialyse de conception très simple en sorte que d'une part il soit susceptible d'être mis en oeuvre directement par le malade à son domicile par exemple, que d'autre part il soit peu couteûx à fabriquer.

La présente invention vise en outre à réaliser un appareil qui soit pour ses constituants essentiels à usage clinique.

A cet effet le dispositif d'hémodialyse selon l'invention comporte un dialyseur dans lequel est ménagée une chambre de circulation de sang en relation par une membrane de type connu avec une chambre de circulation de fluide de dialyse qui entraîne les impuretés du sang transgressant au travers de la membrane, la chambre de circulation du sang étant en relation avec le malade par un conduit de départ et de retour du sang et la chambre de circulation du liquide de dialyse étant en relation avec un réservoir du dit liquide, une cartouche de régénération étant parcourue par le dialysat, suivant une disposition particulièrement avantageuse de l'invention.

Les éléments décrits, dialyseur, réservoir, cartouche et éventuellement conduit à sang et à liquide sont groupés en un même ensemble monobloc susceptible d'être jeté après usage et les éléments constituant les moniteurs d'assistance circulatoire sont indépendants du dit ensemble.

Suivant une autre disposition avantageuse de l'invention le dialyseur et la cartouche de régénération du dialysat constituant avec le réservoir de liquide de dialyse l'ensemble monobloc jetable sont contenus dans le dit réservoir de liquide de dialyse, la circulation du liquide s'effectuant entre eux par le moyen du réservoir lui-même dans le sens cartouche dialyseur et au moyen d'un conduit partiellement extérieur au dit ensemble dans le sens dialyseur cartouche.

Suivant encore une autre disposition de l'invention dans laquelle le sang circule du malade vers le dialyseur et inversement par un conduit et le liquide de dialyse du dialyseur vers la cartouche par un autre conduit, un même moyen de pompage est utilisé pour assurer l'assistance circulatoire des deux fluides (sang et liquide).

L'invention sera mieux comprise par la lecture de la description ci-après d'une forme de réalisation préférentielle donnée à titre d'exemple non limitatif, la dite description étant illustrée par les dessins joints dans lesquels :

- La figure 1 est un schéma de principe représentant un appareil de l'art antérieur.

- la figure 2 est un schéma de principe de l'appareil selon l'invention

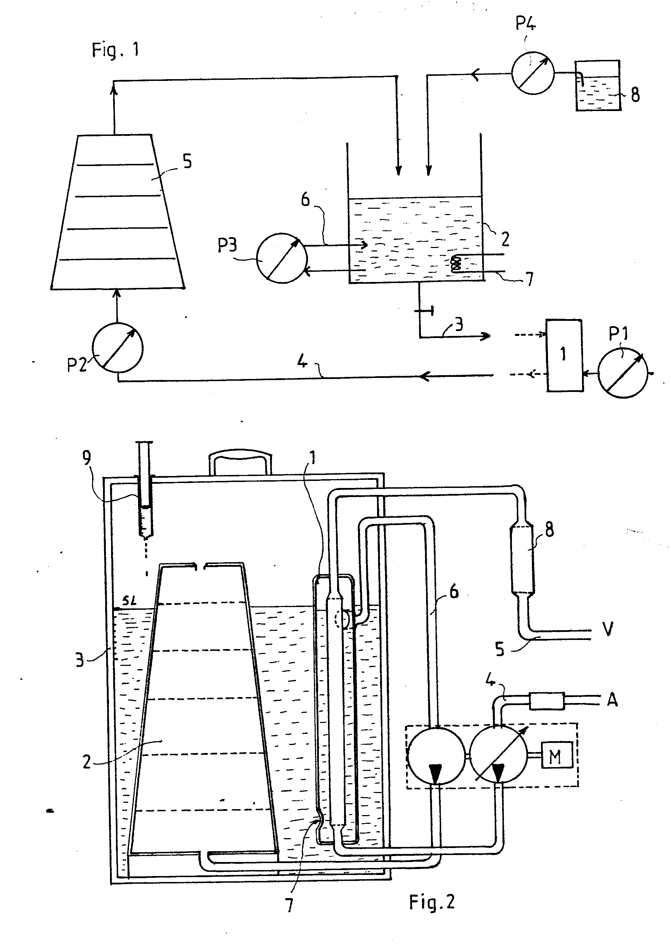

La figure 1 représente de manière schématique un appareil de l'art antérieur du type à cartouche de régénération pour illustrer son principe de fonctionnement.

Cet appareil comporte un dialyseur 1 de type connu en relation avec le malade par un circuit à sang sur lequel est montée à titre de moniteur circulatoire une pompe P1 de type connu.

Le dialyseur est alimenté en liquide de dialyse à partir d'un réservoir 2 par un conduit 3.

Sur le conduit 4 de retour du dialysat au réservoir 2 est montée une pompe P2 intercalée entre le dialyseur et une cartouche régénérante à capacité absorbante 5, qui force le liquide sous une pression donnée à traverse la dite cartouche.

Au niveau du réservoir 2 est monté un circuit 6 de recirculation du liquide avec pompe P3 et un réchauffeur 7 constitué par une résistance électrique.

Afin de maintenir l'équilibre des composants du liquide de dialyse un infusat est distribué en fonction des besoins à partir d'un réservoir 8 au moyen d'une pompe P4.

Les appareils de mesure et de commande ou de contrôle des différents organes tels que décrits ne sont pas représentés pour simplifier le dessin.

On conçoit cependant que ce type de matériel qui comprend au moins quatre pompes plus les appareils de contrôle est relativement complexe et encombrant.

La figure 2 présente un schéma de réalisation de l'appareil d'hémodialyse selon l'invention.

L'appareil selon l'invention est constitué d'un dialyseur 1 de type
connu, d'une cartouche 2 absorbante de régénération du dialysat et
d'un réservoir 3 de liquide de dialyse.

Selon une des caractéristiques essentielles de l'invention, les éléments constitutifs ci-dessus sont groupés en un même ensemble indépendant et séparable des autres éléments constitutifs de l'appareil
ce qui rend le dit ensemble susceptible d'être jeté après usage.

Comme représenté et suivant une autre disposition de l'invention, le
dialyseur 1 et la cartouche 2 sont montés dans le réservoir 3 du
liquide de dialyse, celui-ci constituant un moyen de communication
et de passage du liquide dans le sens cartouche vers dialyseur.
Le montage du dialyseur 1 et de la cartouche 2 dans le réservoir 3
peut être effectué de toute manière connue par exemple au moyen
d'un socle comme il en est représenté un sous la cartouche.

La chambre de circulation du sang du dialyseur 1 reçoit le sang par
un conduit 4 d'arrivée disposé à sa base de préférence et un conduit
5 retourne le sang purifié au malade.
Un conduit 6 à partir du sommet de préférence du dialyseur met en
communication la chambre de passage du liquide de dialyse avec la
base de la cartouche 2 pour évacuer le dialysat vers la dite cartouche en vue de sa régénération.
Un orifice 7 de la base de la chambre de circulation de fluide de
dialyse du dialyseur permet l'alimentation de celui-ci.

0152717

Les conduits 4,5,6 qui sont de préférence souples sont pour partie extérieurs à l'ensemble constitués par le réservoir, le dialyseur et la cartouche.

La communication dans le sens cartouche 2 dialyseur 1 s'effectue par le réservoir 3 et par l'orifice 7.

Le réservoir 4 est un récipient de préférence en matière synthétique moulée de nature telle qu'il ne soit pas altéré par les produits utilisés ni qu'il les altère.

Ce peut être un récipient conçu spécifiquement à cet usage ou un récipient du commerce du type seau à usage alimentaire de forme parallèlèpipèdique dont la contenance peut être de l'ordre de 10 litres.

Ce récipient peut être doté d'un couvercle avec poignée de portage.

Les moniteurs d'assistance circulatoire sont suivant une autre carac téristique disposés et montés extérieurement et/ou de manière indépendante au réservoir 3 et à l'ensemble (1),(2),(3) tel que décrit. Essentiellement ces moniteurs comprennent un moyen de pompage ou pompe P1, un moyen de contrôle de la pression veineuse qui peut par exemple commander l'arrêt et/ou la mise en marche de la pompe P1 et un détecteur d'air.

Suivant une caractéristique essentielle de l'appareil selon l'invention le moyen de pompage ou pompe P1 est utilisé à la fois pour le circuit de sang et pour le circuit de liquide de dialyse.

A cet effet, la pompe P1 est disposée à la fois sur le conduit 4 d'arrivée de sang du malade vers le dialyseur et sur le conduit 6 de retour du dialysat vers la cartouche 2 et le réservoir.

Il va de soi que la pompe P1 peut être de tout type connu adapté à cet usage.

De préférence la pompe P1 est du type volumétrique rotative à galets et à écrasement des conduits 4 et 6 qui sont des conduits souples, la dite pompe étant adaptée au niveau de son chariot de roulement pour recevoir deux raccords de pompe et deux conduits.

Il peut par exemple être fait usage d'une pompe de marque "WATSON-MARLOW" de type "MHRE 200"

A la pompe est associé un manomètre avec alarmes électriques qui commande l'arrêt de toute manière connue en cas de pression veineuse supérieure ou inférieure à la valeur de pression souhaitable.

Sur le conduit 5 de retour du sang vers le malade est monté un détecteur d'air 8.

Le détecteur d'air est constitué d'un piège à bulles de type connu qui permet de vérifier visuellement la présence d'air et d'arrêter la pompe dans le cas de baisse du niveau du piège à bulles révélatrice du passage d'air dans le circuit du sang.

Il peut être associé à une sonde qui contrôle la présence d'air par exemple au moyen d'une cellule photoélectrique stimulée par la présence d'air en sorte d'arrêter la pompe.

Les moniteurs d'assistance circulatoire peuvent être fixes de toute manière connue latéralement à sa paroi extérieure ou dans un logement de celle-ci.

Dans le réservoir 3 sur une de ses parois latérales internes ou sur le couvercle est monté un dispositif 9 constitué par exemple par une seringue permettant de distribuer manuellement un infusat de produits adjuvants pour maintenir l'équilibre du liquide de dialyse.

Il va de soi que cette fonction peut également être remplie par une seringue de capacité appropriée que l'utilisateur peut prévoir.

L'utilisation de l'appareil selon l'invention est particulièrement simple.

Après ouverture du réservoir l'utilisateur qui peut être le malade y verse le produit concentré et l'eau chaude permettant d'obtenir le liquide de dialyse.

Il prépare l'infusat qui sera utilisé au fur et à mesure du déroulement de l'opération.

La température du liquide de dialyse peut être maintenue à la valeur de 38° à 40° en plaçant le réservoir 3 dans une enveloppe isotherme du type glacière du commerce.

Après raccordement du circuit sanguin et amorçage par du serum physiologique du circuit sanguin et du circuit dialysat l'opération peut se dérouler.

Le dialysat après passage dans le dialyseur sort de la cartouche et maintient un niveau constant dans le récipient où son apport régulier contribue à un brassage et à une alimentation en circuit fermé.

Au terme de l'opération, l'utilisateur prélève les éléments constituant les moniteurs d'assistance circulatoire qu'il conserve pour un autre usage.

Les autres éléments, réservoir, cartouche, dialyseur, conduits, peuvent être jetés.

L'invention permet de réaliser un appareil simple et peu coûteux qui peut être mis en oeuvre par le malade lui-même avec un minimum d'informations.

L'invention peut recevoir des aménagements et des variantes dans le domaine des équivalents techniques sans pour autant sortir du cadre du présent brevet.

REVENDICATIONS

1. Appareil d'hémodialyse comportant un dialyseur (1) dans lequel est ménagé une chambre de circulation de sang en relation par une membrane perméable ou semi-perméable, de type connu avec une chambre de circulation de liquide de dialyse, dans le dit dialyseur, qui entraîne les impuretés transgressant au travers de la membrane, la chambre de circulation du sang étant en relation avec le malade par un conduit d'arrivée (4) et de retour (5) du sang et la chambre de circulation du liquide de dialyse étant en relation avec un réservoir (3) du dit liquide, une cartouche (2) de régénération étant traversée par le dialysat à son retour vers le réservoir (3) le dit appareil étant caractérisé en ce que le dialyseur (1), la cartouche (2) et le réservoir (3) sont groupés en un même ensemble susceptible d'être jeté après usage et que les éléments constituant les moniteurs d'assistance circulatoire tels que organes de pompage. et moyens de contrôle sont indépendants et séparables du dit ensemble.

2. Appareil selon la revendication 1 caractérisé en ce que le dialyseur (1) et la cartouche (2) de régénération constituant avec le réservoir (3) de liquide de dialyse l'ensemble jetable sont contenus dans le dit réservoir.

3. Appareil selon la revendication 2 caractérisé en ce que la circulation du liquide de dialyse dans le sens cartouche (2) dialyseur (1) s'effectue par le moyen du réservoir et par un conduit (6) partiellement extérieur au réservoir dans le sens dialyseur - cartouche.

4. Appareil selon la revendication 1 dans lequel le sang circule du malade vers le dialyseur (1) par un conduit (4) et inversement par un conduit (5) et le liquide de dialyse du dialyseur (1) vers la cartouche (2) et le réservoir (3) par au moins un conduit (6) caractérisé en ce qu'un même moyen de pompage est utilisé pour l'assistance circulatoire des deux liquides.

PL. Unique

0152717

Fig. 1

Fig. 2

Office européen
des brevets

**RAPPORT DE RECHERCHE EUROPEENNE**

Numéro de la demande

EP 84 45 0024

| | **DOCUMENTS CONSIDERES COMME PERTINENTS** | | |
|---|---|---|---|
| Catégorie | Citation du document avec indication, en cas de besoin, des parties pertinentes | Revendication concernee | CLASSEMENT DE LA DEMANDE (Int. Cl.4) |
| D,Y | FR-A-2 243 704 (TAKEDA)<br>* en entier, en particulier figures 6,7; page 2, ligne 38 - page 3, ligne 4; page 13, lignes 7-9,13-15,19-28 * | 1-3 | A 61 M 1/36 |
| Y | US-A-3 650 404 (VERSACI)<br>* en entier, en particulier figure 1; résumé * | 1-3 | |
| Y | US-A-4 153 554 (VON DER HEIDE)<br>* colonne 13, ligne 21 - colonne 14, ligne 2; figure 13; résumé * | 1-3 | |
| A | GB-A-2 009 863 (LEHNHOFF)<br>* page 1, lignes 69-77 * | 4 | |
| | ----- | | DOMAINES TECHNIQUES RECHERCHES (Int. Cl.4)<br><br>A 61 M |

Le present rapport de recherche a été établi pour toutes les revendications

| Lieu de la recherche<br>LA HAYE | Date d'achèvement de la recherche<br>28-01-1985 | Examinateur<br>VEREECKE A. |
|---|---|---|